# EUROPEAN PATENT APPLICATION

(11) **EP 1 504 774 A1**
(43) Date of publication of application: **09.02.2005**
(21) Application number: 02724786.5
(22) Date of filing: 14.05.2002
(51) Int. Cl.: A61L 27/24, A61L 27/56, A61L 27/58, A61L 27/60, A61L 27/44

(54) **ARTIFICIAL EXTRACELLULAR MATRIX AND PROCESS FOR PRODUCING THE SAME**

(71) Applicant: Hokkaido Technology Licensing Office Co., Ltd., Sapporo-Shi, Hokkaido 060-0807 (JP)
(72) Inventor: TAKAI, Mitsuo, Sapporo-shi, Hokkaido 064-0917 (JP); ERATA, Tomoki, Sapporo-shi, Hokkaido 001-0922 (JP); YUNOKI, Shunji, Sapporo-shi, Hokkaido 060-0006 (JP); FUJITA, Tatsuya, Sapporo-shi, Hokkaido 005-0821 (JP)
(74) Representative: Curtis, Philip Anthony
(86) International application number: PCT/JP2002/004665
(87) International publication number: WO 2003/094985

(57) **Abstract**

The artificial extracellular matrix of the present invention comprises heat stable fish collagen produced from fish collagen with a heat denaturation temperature lower than 37°C by improving its heat stability. The artificial extracellular matrix of the invention has an adequate stability in living bodies and a function to activate tissue repair at a high level and requires no addition of heat denatured collagen. In addition, the artificial extracellular matrix is unlikely to contain pathogenic organisms because it uses collagen originated from fish.

## Description

### Technical Field

The present invention relates to artificial extracellular matrix and a process for producing the same. The invention in particular relates to artificial extracellular matrix that have an adequate stability in living bodies and a function to activate tissue repair at a high level, that require no addition of heat denatured collagen, and that are unlikely to be affected by pathogens, and a process for producing them.

### Background Art

It is a quite important issue, when organs or tissues are injured or impaired due to trauma or illnesses, to regenerate such organs and tissues to restore function. Medical treatment to regenerate such organs and tissues is referred to as tissue engineering, and has been extensively studied for its development.

Some approaches are known to tissue engineering. A first approach involves constructing organs or tissues outside the patient's body and replacing them into the body, and a second approach involves providing a foothold for organ or tissue regeneration for the defective part of the organ or tissue, thus enhancing active organ or tissue regeneration.

Either method requires an artificial extracellular matrix that will serve as a foothold for organ or tissue regeneration. The effect to promote tissue repair is a requirement for such artificial extracellular matrix. Tissue engineering for the skin will be described.

As is well known, the skin is composed of three-layer structure consisting of epidermis, dermis and subcutaneous tissue, and when the entire dermis is destroyed due to burn and so forth, the skin would become keloid because the skin is not regenerated. Therefore, skin grafting is now done as a superior means of repairing such destroyed wounded surface. Although autologous skin grafting is preferable, the procedure is not possible when a wide skin area has been impaired. In these cases, in the case of identical twins, grafting of skin from one to the other has been done, but otherwise it is difficult.

To resolve these problems, a method of transplanting an artificial extracellular matrix onto the defective skin part has been attempted as an alternative method to promote healing. Such artificial extracellular matrix are generally referred to as artificial skin. Examples of artificial skin include commercialized artificial skin products using natural polymers such as collagen sheets and chitin nonwoven fabric.

However, because such artificial skin materials are highly biodegradable, they sometimes disappear even before being transformed into autologous tissue, or remain behind in the body as foreign matter due to excessive crosslinking. In addition, they are also insufficient in terms of activating tissue repair.

Therefore, artificial skin has been developed which has adequate in vivo stability and has an improved ability to activate tissue repair. For example, Japanese Patent Publication No. 6-18583 discloses artificial skin having wound contact layers comprising artificial extracellular matrix consisting of fibrillated collagen and denatured collagen (gelatine) that were subjected to crosslinking reaction by dehydration heat crosslinking reaction. The artificial skin disclosed in the patent publication has adequate in vivo stability but insufficient ability to activate tissue repair. In addition, the artificial skin disclosed in the patent publication undergoes almost no heat denaturation at body temperatures because mammalian collagen used has high heat denaturation temperatures and is even subjectedtocrosslinkingreaction. Consequently, it was necessary to add heat denatured collagen (gelatine) commonly said to have the ability to promote the adhesion and migration of fibroblasts, or to denature part of the aforementioned collagen by heating.

The above problem applies also to the artificial extracellular matrix used for constructing implantable artificial organs such as artificial liver. In other words, the artificial extracellular matrix shape must be retained in order for the autologous cells to enter and reconstruct the defective organ part, and it is important to promote entry of autologous cells and tissue regeneration.

Moreover, the above property requirement also applies to a case where artificial extracellular matrix are seeded with a variety of cells and incubated to construct organs and tissues in vitro. For example, Japanese Patent Laid-Open No. 11-47258 discloses a medical basic material comprising a mixture of gelatine and collagen that was irradiated with ultraviolet rays to produce crosslinking. However, the medical basic material disclosed in the patent publication also required adding heat denatured collagen (gelatine) because it used collagen of mammalian origin that was subjected to crosslinking reaction. In addition, the material was insufficient in terms of promoting entry of autologous cells and tissue regeneration. Furthermore, cattle and pigs are used as main sources of collagen. Because cattle and pigs are marketed as domestic animals, their supplies may be stopped when there is a disease outbreak, or humans may contract pathogenic organisms through the use of medical basic materials produced from collagen from cattle and pigs.

Thus it is an object of the present invention to provide artificial extracellularmatrix that have an adequate stability in living bodies and a function to activate tissue repair at a high level, that require no addition of heat denatured collagen, and that are unlikely to be affected by pathogens. It is another object of the invention to provide a process for producing the aforementioned artificial extracellular matrix.

### Disclosure of the Invention

The invention has been completed as a result of intensive studies conducted to achieve the above objects, which have resulted in findings that by improving the heat stability of fish collagen with a heat denaturation temperature lower than 37°C, it will be possible to obtain artificial extracellular matrix that achieve the above objects.

The invention has been based on the above findings and provides artificial extracellular matrix containing heat stable fish collagenproduced from fish collagen with a heat denaturation temperature lower than 37°C by improving its heat stability.

The term "heat stability" as used herein means the susceptibility of collagen to heat denaturation when allowed to stand in a humid environment at 37°C. When heated colloagen under humid condition, the triple-stranded helical structure of collagen consisting of three polypeptide chains of about 100,000 in molecular weight is broken, resulting in random-coil-shaped gelatine. The temperature at which a structural change occurs is generally referred to as the denaturation temperature, and is considered to correspond to the environmental temperature of the organism from which the collagen is derived. In the case of homeothermal animals such as mammals and birds, the denaturation temperature of collagen is around 37°C, a temperature representing their body temperature. In the case of fish living in low-temperature sea areas such as the northern sea, the denaturation temperature of collagen is as low as 10 to 20°C, representing a much lower temperature than those for homeothermal animals.

The invention provides artificial extracellular matrix comprising fish collagen with a heat denaturation rate of 20 to 90%.

The process for producing the artificial extracellular matrix according to the present invention is a process for producing the artificial extracellular matrix involving the step of molding a solution that contains fish collagen, and is characterized in that it includes the step of improving the heat stability of fish collagen with a heat denaturation temperature lower than 37°C.

The present invention includes the use of the above artificial extracellular matrix of the invention for the regeneration of organs or tissues in animals.

In addition, the method of regenerating an organ or tissue in animals of the invention comprises transplanting the above artificial extracellular matrix of the invention onto the defective part of the organ or tissue.

### Brief Description of the Drawings

Figure 1 is a photograph showing the result of a subcutaneous graft experiment using the artificial extracellular matrix of the present invention; and
Figure 2 is a photograph showing the result of a subcutaneous graft experiment using a conventional artificial extracellular matrix.

### Best Mode for Carrying Out the Invention

Hereinafter, the artificial extracellular matrix of the invention will be described.

The artificial extracellular matrix of the invention comprises heat stable fish collagen produced from fish collagen with a denaturation temperature lower than 37°C by improving its heat stability.

The fish species used for deriving collagen for the production of the artificial extracellular matrix of the present invention are not limited to particular species. Concerning the collagen used in the present invention, what counts is its property and not the fish species used to derive the collagen. However, using rare fish species is not preferable because stable supply of collagen is unsecured. In addition, considering that a large quantity of fish skin generated during processing has been disposed of as fishery waste, it is desirable to use such fish skin in light of utilizing waste as resources.

Although any fish collagen may be used for producing the artificial extracellular matrix of the invention, preferably type I collagen derived from fish skin or a mixture of various types of collagen consisting primarily of type I collagen is used in view of yield and heat stability. In addition, the effectiveness of the present invention may not be elicited sufficiently if the heat stability of the collagen used before applying heat stability treatment is too high. Concretely, the heat denaturation temperature obtained from the change in optical rotatory power with temperature (measured according to Kimura et al., Biosci. Biotech. Biochem., 60(12), 2092-2094, 1996) must be lower than the body temperature of 37°C. In addition, the heat denaturation temperature is preferably 35°C or lower, more preferably 30°C or lower, yet more preferably 25°C or lower, and most preferably 20°C or lower.

The methods for improving the heat stability of the fish collagen contained in the artificial extracellular matrix of the invention are not limited to particular methods, and include various chemical and physical crosslinking methods. These methods will be described herein later.

"Heat stability of fish collagen" as used herein can be determined as a heat denaturation rate. The heat denaturation rate can be determined by immersing fish collagen in a sufficient volume of phosphate buffer at 37°C for 72 hours and determining the concentration of gelatine eluted.

Concretely, the heat denaturation rate (%) can be measured by the following procedure.

An artificial extracellular matrix was placed in a desiccator containing phosphorus pentaoxide, vacuum dried at room temperature for three days, and about 20 mg of the dried artificial extracellular matrix was weighed accurately and used as a specimen. The accurately weighed specimen was immersed in 20 ml of phosphate buffer and incubated at 37°C for 72 hours in an incubator. The container was tightly closed to prevent evaporation of the specimen.

After standing for 72 hours, the phosphate buffer in the container was filtered through a membrane filter having a pore size of 0.45 µm to prepare a specimen for the measurement of denaturation rates.

Separately, an aqueous solution of collagen was heated at 60°C for one hour to prepare a solution of heat denatured collagen (gelatine), the absorbance of this gelatine solution was measured at 230 nm, and gelatine solutions of different concentrations were prepared to generate a working curve.

The specimen for the measurement of denaturation rates prepared as above was analyzed for absorbance at 230 nm, the gelatine concentration was determined from the working curve, and the heat denaturation rate (%) was calculated from the gelatine concentration and the specimen weight. Because the working curve is linear only when the gelatine concentration is up to about 0.3 mg/ml, if collagen dissolves to a great extent resulting in a departure of the gelatine concentration in the specimen from the working curve, the specimen was diluted before measurement and the gelatine concentration was calculated from the dilution ratio to determine the heat denaturation rate.

The artificial extracellular matrix of the present invention contains fish collagen with a heat denaturation rate of 20 to 90%, preferably 20 to 80%, and more preferably 20 to 70%. It is preferable to use fish collagen with a heat denaturation rate of 20 to 90%, because heat denaturation rates lower than 20% are unsuitable for the adhesion, migration and proliferation of fibroblasts, and those higher than 90% are unsuitable for maintaining shape required for artificial extracellular matrix.

Physicochemical properties of fish collagen may be modified using known chemical techniques as long as the effectiveness of the invention is not impaired. These chemical modifications include methylation, succinylation and acetylation. In addition, as known in the art, a collagen molecule has at its both ends a peptide structure lacking helical structure called telopeptide. A telopeptide has 12 to 27 amino acid residues, and the antigenicity of collagen is said to be expressed by this site. It therefore is preferable to remove this telopeptide site when producing the artificial extracellular matrix of the present invention. Removal of the telopeptide site can be accomplished using proteolytic enzymes such as pepsin.

Other natural polymers and cell growth factors may be added to the artificial extracellular matrix of the present invention as long as it does not impair the effectiveness of the present invention. The natural polymers include alginic acid, hyaluronic acid, chondroitin sulfate, chitinandchitosan. In addition, the cell growth factors include fibroblast growth factor (FGF), hepatocyte growth factor (HGF), vascular endothelial growth factor (VEGF).

Preferably, the artificial extracellular matrix of the invention is of sponge-like porous structure. The thickness and shape of such artificial extracellular matrix can vary according to the condition of the targeted affected part, with the thickness preferably varying from about 0.3 to 30 mm and the shape varying from thin sheets and plates to rods, spheres, spindles and bilaterally convex lenses. Moreover, the pore size of the sponge-like structure can be varied according to the condition of the targeted affected part, and can be controlled by adjusting the concentration of collagen solution at production and the freezing temperature.

The artificial extracellular matrix of the invention may be laminated with substrates. A substrate means sheet material laminated to confer strength, moisture permeability, antibacterial activity, etc. on the artificial extracellular matrix. The method of laminating substrates with artificial extracellular matrix for this purpose is well known in the art, and materials known in the art may be used for the artificial extracellular matrix of the present invention. Such materials include films, knits and nonwoven fabrics produced from synthetic polymers such as nylon and silicon and natural polymers such as silk, cotton, chitin and chitosan. Preferably, such substrates are laminated on one of the plane surfaces if the shape of the artificial extracellular matrix is flat, and on the entire surface if it is mass-like.

The process for producing the artificial extracellular matrix of the invention will be described later.

The artificial extracellular matrix of the invention will be used as follows.

Organ or tissue parts constructed in vitro are transplanted onto the defective part of the organ or tissue of the body. Concretely, the artificial extracellular matrix is seeded with autologous or heterologous cells and incubated before transplantation to achieve some degree of organ or tissue repair. The cell types used for seeding and incubation may be selected from those derived from the organ or tissue to which the artificial extracellular matrix of the present invention is applied. For example, fibroblasts are used for seeding and incubation when the artificial extracellular matrix of the present invention is applied to the skin, and hepatic cells are used when it is applied to the liver.

When seeding the artificial extracellular matrix of the present invention with autologous or heterologous cells, preferably the density of the autologous or heterologous cells is about 10,000 cells/cm². Thus, the artificial extracellular matrix seeded with autologous or heterologous cells is incubated in a 5% CO₂ incubator at 37°C for one to two weeks before use.

In addition, the artificial extracellular matrix of the invention is used to provide a foothold for regeneration at the defective part of the organ or tissue of the body. In these cases, instead of seeding/incubation, the artificial extracellular matrix of the invention is directly transplanted onto the defective part of the organ or tissue of the body. The artificial extracellular matrix of the invention is molded in accordance with the shape of the defective transplantation site.

As described above, it is possible to regenerate organs or tissues in animals by transplanting the artificial extracellular matrix of the invention onto the defective part of the organ or tissue.

In addition, the artificial extracellular matrix of the present invention can be used as a foothold for entry by various cells or as a carrier for the culture of various cells. In short, the artificial extracellular matrix of the invention can be used as the matrix for artificial organs. Preferably, the artificial extracellular matrix of the present invention is used as a matrix for artificial organs for the skin, cartilage, bone, vessels, cornea, liver, etc., in light of utilizing an improved heat stability of collagen that the invention matrix has. More preferably it is used as a matrix for artificial organs for the skin, cartilage and bone. Most preferably it is used as a matrix for artificial skin.

The process for producing method for the artificial extracellular matrix of the invention will be described below.

The process for producing artificial extracellular matrix according to the present invention involves the step of molding a solution that contains fish collagen, and is characterized in that it includes the step of improving the heat stability of fish collagen with a heat denaturation temperature of below 37°C.

As the fish collagen used in the producing process for the artificial extracellular matrix of the present invention, those collagens as described in the above sections on the artificial extracellular matrix of the invention may be used. Fish collagen used in the process for producing artificial extracellular matrix of the invention has a heat denaturation temperature of lower than 37°C. The heat denaturation temperature is preferably 35°C or lower, more preferably 30°C or lower, yet more preferably 25°C or lower, and most preferably 20°C or lower.

The producing process for the artificial extracellular matrix of the invention involves the step of molding a solution containing fish collagen. Any well known conventional method of molding a solution containing collagen can be used without any particular restriction as the method of molding a solution containing fish collagen. These conventional methods include the following methods 1 to 5.
1. Fish collagen solution poured into a mold is air-dried or freeze-dried.
2. Fish collagen solution poured into a mold is gelatinized by a known method then air-dried or freeze-dried.
3. Fish collagen solution poured into a mold along with various cells is gelatinized (method described in Human Cell, 1(2), 150-161, 1988).
4. Fish collagen solution is processed into fibers using a known method, which are then molded into sheets.
5. Fish collagen solution poured into a mold or fish collagen solution extruded from a die is solidified in solidification phase.

Preferably, molding methods 1, 2 and 5, and method 1 in particular, are used. to achieve satisfactory invasion of fibroblasts.

The concentration of fish collagen in the solution containing fish collagen used is preferably about 0.1 to 2.0% by mass. Any solvent at a pH range of 1 to 7 can be used without any particular restriction, and, for example, water, various organic acids such as dilute acetic acid, and inorganic acid aqueous solutions may be used.

Other natural polymers and cell growth factors may be added to the above solution containing fish collagen as long as it does not impair the effectiveness of the present invention. The natural polymers include alginic acid, hyaluronic acid, chondroitin sulfate, chitin and chitosan. In addition, the cell growth factors include fibroblast growth factor (FGF), hepatocyte growth factor (HGF), and vascular endothelial growth factor (VEGF).

Moreover, air bubbles may be produced in the solution containing fish collagen by homogenization before molding to produce a sponge-like artificial extracellular matrix.

The methods for improving the heat stability of fish collagen used in the producing process for theartificialextracellularmatrixoftheinvention include various crosslinking reaction such as chemical and physical crosslinking methods. Heat stability of fish collagen can be improved by applying crosslinking reaction thereto.

The above chemical crosslinking methods include known methods using aldehyde crosslinking agents, ethylene glycol diglycidyl ether and isocyanate crosslinking agents. The above physical crosslinking methods include ultraviolet crosslinking methods, gamma-ray crosslinking methods and dehydration heat crosslinking methods. Where the crosslinking agent used in chemical crosslinking reaction remains behind after crosslinking to exhibit cytotoxicity, or remains in the body as foreign matter due to poor biodegradability, physical crosslinking reaction is preferably used which incurs no such problem. Particularly ultraviolet crosslinking method among others is preferably used considering ease of handling.

In the process for producing the artificial extracellular matrix of the invention, heat stability may be improved either before or after molding fish collagen.

When heat stability is improved before molding, it can be carried out by adding any of the above crosslinking agents to the solution containing fish collagen. In the case of physical crosslinking reaction, the solution containing fish collagen is irradiated with ultraviolet rays and so forth while the solution is agitated or allowed to stand. When heat stability is improved after molding using physical crosslinking reaction, if the artificial extracellular matrix is film-shaped, the matrix is preferably irradiated with ultraviolet rays and so forth on both sides. If the matrix is a mass, preferably the entire surface is irradiated in a uniform manner. When fish collagen is subjected to crosslinking through ultraviolet irradiation to improve its heat stability, preferably the ultraviolet wavelength is approximately 250 to 270 nm, and its intensity is approximately 0.1 to 1.0 mW/cm².

In the process for producing the artificial extracellular matrix of the invention, the artificial extracellular matrix may be laminated with substrates. A substrate means as described above sheet material laminated to confer strength, moisture permeability, antibacterial activity, etc. on the artificial extracellular matrix. Materials used as a substrate include those mentioned previously. Methods for laminating substrates with artificial extracellular matrix include a method that involves pouring a solution containing fish collagen into substrates followed by air-drying then freeze-drying or gelatinization, and a method that involves bonding substrates to artificial extracellular matrix after molding. Various adhesives may be used for bonding in the latter method, or a solution containing fish collagen may be applied onto the contact surfaces of substrates or artificial extracellular matrix before laminating the substrates and drying to fix them.

### Examples

The present invention will be described in more detail below with reference to examples. Obviously, the scope of the present invention is not limited by these examples.

### Example 1

Salmon (Oncorhynchus keta) skin was degreased with methanol and chloroform, and 130 g of the degreased skin was immersed in 3 liters of 0.5 M acetic acid solution at 4°C for three days to extract collagen. Subsequently, the swollen salmon skin was filtered out by gauze, and the filtrate was centrifuged at 10, 000 g, 4°C for 30 minutes to remove precipitating salmon skin debris.

30 mg of pepsin was added to the supernatant, which was maintained at 4°C while agitating for two days to digest collagen with pepsin. After digestion with pepsin, salting out was done in 5% sodium chloride solution for 24 hours, and the resultant solution was centrifuged at 10, 000 g, 4°C for 30 minutes. The supernatant was discarded, and collagen residue was collected. 0.5 M acetic acid solution was added to the collagen residue collected to dissolve at 4°C for two days with agitation, resulting in a collagen solution. The cycle of salting out, centrifugal separation and dissolution of collagen was repeated twice. The collagen solution (in 0.5 M acetic acid solution) obtained was centrifuged at 100, 000 g, 4°C for one hour using an ultracentrifuge to remove minute impurities. The supernatant was placed in a dialysis membrane to dialyze in deionized water, and the resultant totally neutralized collagen solution was freeze-dried. The heat denaturation temperature of the collagen obtained was 19°C.

The freeze-dried collagen thus obtained was added to dilute hydrochloric acid at pH 3.0 so as to give a concentration of 0.5% by mass, and was dissolved at 4°C for three days with agitation to make collagen solution. The collagen solution was poured into a plastic mold such that the thickness after drying would be approximately 0.5 cm and was frozen with liquid nitrogen then freeze-dried. A freeze-dried collagen sponge (0.5 cm thick) was placed in a desiccator containing phosphorus pentaoxide then vacuum dried at room temperature for three days.

Toperformultravioletcrosslinkingtoimprove the heat stability of collagen, the vacuum dried collagen sponge was placed under an ultraviolet lamp of 254 nm wavelength for nine hours such that the ultraviolet intensity would be 0.65 mW/cm², and the artificial extracellular matrix of the present invention was produced.

Subsequently, the heat stability of collagen contained in the artificial extracellular matrix was measured by the following method.

An artificial extracellular matrix was placed in a desiccator containing phosphorus pentaoxide, vacuum dried at room temperature for three days, and about 20 mg of the dried artificial extracellular matrix was weighed accurately and used as a specimen. The accurately weighed specimen was immersed in 20 ml of phosphate buffer and incubated at 37°C for 72 hours in an incubator. The container was tightly closed to prevent evaporation of the specimen.

After standing for 72 hours, the phosphate buffer in the container was filtered through a membrane filter having a pore size of 0.45 µm to prepare a specimen for the measurement o'f denaturation rates.

Separately, an aqueous solution of collagen was heated at 60°C for one hour to prepare a solution of heat denatured collagen (gelatine), the absorbance of this gelatine solution was measured at 230 nm, and gelatine solutions of different concentrations were prepared to generate a working curve.

The specimen for the measurement of denaturation rates prepared as above was diluted two-fold and analyzed for absorbance at 230 nm, the gelatine concentration determined from the working curve was doubled to find the actual gelatine concentration, and the heat denaturation rate (%) was calculated from the gelatine concentration and the specimen weight.

The heat denaturation rate of the artificial extracellular matrix of Example 1 was 52%.

A subcutaneous graft experiment using the artificial extracellular matrix obtained was conducted according to the procedure below.

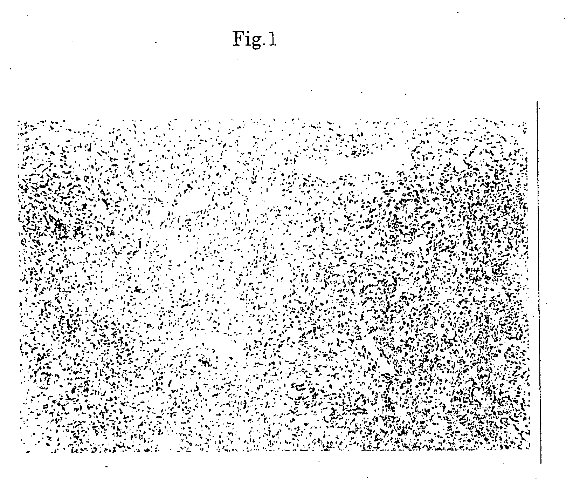

ABalb/cmouse (female) was used as a test animal. After anesthetizing a mouse with ether, the back part was shaved using depilation foam, and an incision of about 1 cm was made at the median back part to form a pocket in the areolar tissue under the cutaneous muscle. The artificial extracellular matrix made as above was transplanted into this pocket, which was sutured with nylon thread. The artificial extracellular matrix was subjected to biopsy 14 days after transplantation, which involved immersing the specimen in 10% neutral buffer formalin solution overnight for fixation before histopathology. For histopathological stain, hematoxylin-eosin (H-E) stain, Masson's trichrome stain and anti-CD31 stain were used. The results are shown in Figure 1.

As shown in Figure 1, so-called granulation tissues characterized by vascular hyperplasia and prevailing fibroblasts, lymphocytes and histiocytes were formed in the sponge. In addition, anti-CD31 stain revealed endodermic components and lumen formation.

### Comparative Example 1

The same operations as those in Example 1 were followed to obtain an artificial extracellular matrix except that ultraviolet crosslinking reaction was omitted. The heat denaturation rate of the artificial extracellular matrix obtained was 100% when determined using the same procedure as Example 1. A heat denaturation rate of 100% means that when the sample artificial extracellular matrix is subjected to a subcutaneous transplantation test, it undergoes complete heat denaturation within three days and dissolves into tissues.

The artificial extracellular matrix produced as above was subjected to a subcutaneous transplantation test as in Example 1. The transplanted artificial extracellular matrix disappeared completely seven days after transplantation.

### Comparative Example 2

Using "Terudermis Skin Defect Graft" (Terumo Corporation), the heat denaturation rate was determined as in Example 1 and a subcutaneous transplantation test was conducted. Terudermis Skin Defect Graft is made from bovine (mammal) collagen, which is subjected to dehydration heat crosslinking, and is supplemented with heat denatured collagen. A specimen from the product was immersed in dilute hydrochloric acid at pH 3.0, heated at 100°C for 10 minutes until complete dissolution, and the content of collagen and heat denatured collagen in the specimen, which was determined by the same method as that used in the measurement of heat denaturation rates, was 93%. The remaining 7% is regarded as minerals. Therefore, the heat denaturation rate was calculated to be 15% based on the collagen content of 93%.

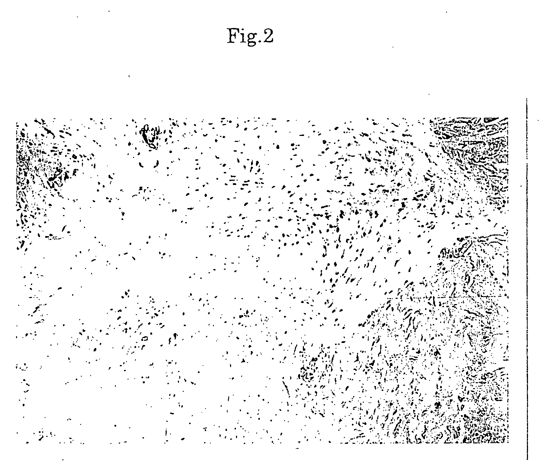

A .subcutaneous transplantation test was conducted in the same way as in Example 1, but the laminated silicone sheet was removed from "Terudermis Skin Defect Graft" in Comparative Example 2. The results are shown in Figure 2. As shown in Figure 2, no vascular hyperplasia was observed 14 days after transplantation, clearly indicating poor cellular components.

Findings from Example 1, Comparative Example 1 and Comparative Example 2 are summarized in Table 1 below.

**Table 1**

| | Artificial extracellular matrix | | | | Changes in tissues 14 days after transplantation | |
|---|---|---|---|---|---|---|
| | Origin | Heat stabilization method | Addition of heat denatured collagen | Heat denaturation rate (%) | Vascular hyperplasia | Cell invasion |
| Example 1 | Salmon (fish) | UV crosslinking | None | 52 | Present | Present |
| Comparative Example 1 | Salmon (fish) | - | None | 100 | - | - |
| Comparative Example 2 | Bovine (mammal) | Dehydration heat crosslinking | Yes | 15 | Absent | Poor |

As described in detail above, the artificial extracellular matrix of the invention has adequate stability in living bodies and the function to activate tissue repair at a high level and requires no addition of heat denatured collagen. In addition, the artificial extracellular matrix is unlikely to contain pathogenic organisms because it uses collagen derived from fish.

The above described artificial extracellular matrix can be easily produced by the process for producing the artificial extracellular matrix of the present invention.

## Claims

1. An artificial extracellular matrix comprising heat stable fish collagen produced from fish collagen with a heat denaturation temperature lower than 37°C by improving the heat stability thereof.

2. An artificial extracellular matrix comprising fish collagen with a heat denaturation rate of 20 to 90%.

3. The artificial extracellular matrix according to claim 1 or 2 wherein the fish collagen has been subjected to crosslinking reaction.

4. The artificial extracellular matrix according to claim 3 wherein the fish collagen has been subjected to crosslinking reaction by ultraviolet irradiation.

5. The artificial extracellular matrix according to any one of claims 1 to 4 wherein the artificial extracellular matrix is used as a matrix for artificial skin.

6. The artificial extracellular matrix according to any one of claims 1 to 5 wherein the artificial extracellular matrix is of sponge type.

7. The artificial extracellular matrix according to any one of claims 1 to 6 wherein the artificial extracellular matrix is laminated on a substrate.

8. The artificial extracellular matrix according to any one of claims 1 to 7 wherein the artificial matrix is incubated with seeded autologous or heterologous cells.

9. A process for producing an artificial extracellular matrix comprising the step of molding a solution containing fish collagen **characterized in that** the method includes the step of improving the heat stability of fish collagen with a heat denaturation temperature lower than 37°C.

10. The process for producing an artificial extracellular matrix according to claim 9 wherein the fish collagen is subjected to crosslinking reaction to improve its heat stability.

11. The process for producing an artificial extracellular matrix according to claim 10 wherein the fish collagen is subjected to crosslinking reaction by ultraviolet irradiation.

12. The process for producing an artificial extracellular matrix according to any one of claims 9 to 11 comprising the step of laminating the artificial extracellular matrix on a substrate.

13. A use of the artificial extracellular matrix according to any one of claims 1 to 8 for the regeneration of an organ or tissue in an animal.

14. A method for regenerating an organ or tissue in an animal comprising transplanting an artificial extracellular matrix as recited in any one of claims 1 to 8 onto the defective part of the organ or tissue.
